# FASCICULE DE BREVET EUROPEEN

(11) **EP 0 539 270 B1**
(45) Date de publication et mention de la délivrance du brevet: **17.01.1996**
(21) Numéro de dépôt: 92402817.8
(22) Date de dépôt: 15.10.1992
(51) Int. Cl.: C07C 2/76, B01J 19/24

(54) **Procédé de conversion thermique du méthane et réacteur pour la mise en oeuvre du procédé**
Verfahren zur thermischen Konvertierung von Methan und Reaktor zur Durchführung des Prozesses
Process for the thermal conversion of methane and reactor for this process

(30) Priorité: 17.10.1991 FR 9112948
(43) Date de publication de la demande: 28.04.1993
(73) Titulaire: INSTITUT FRANCAIS DU PETROLE, F-92502 Rueil-Malmaison (FR); GAZ DE FRANCE, F-75017 Paris (FR); ELECTRICITE DE FRANCE, F-75384 Paris Cedex 08 (FR)
(72) Inventeur: Weill, Jérôme, F-69005 Lyon (FR); Capogna, Laure, F-93200 Saint Denis (FR); Arrondel, Véronique, F-78800 Houilles (FR); Boumendil, Jean-Jack, F-06480 La Colle sur Loup (FR)
(74) Mandataire: Andreeff, François

(56) Documents cités:
- EP-A- 0 323 287
- EP-A- 0 457 643

## Description

L'invention concerne un procédé de conversion thermique du méthane en hydrocarbures de poids moléculaires plus élevés et le dispositif pour la mise en oeuvre de ce procédé. Elle concerne plus particulièrement un procédé de conversion ou craquage thermique du méthane dans un réacteur comportant des moyens de chauffage électrique et permettant, par couplage thermique déshydrogénant de cette molécule, la production, principalement, d'acétylène, d'éthylène, de benzène et d'un peu de coke.

Toutes les sources de méthane bien connues de l'homme du métier peuvent être utilisées. Comme source de méthane très courante, on peut citer le gaz naturel. Une liste non exhaustive de ces sources a par exemple été fournie dans la demande de brevet européen au nom de la demanderesse EP-A-323287. Dans la majorité des cas, le gaz contenant du méthane que l'on introduit dans le réacteur contient de 1 à 90 % d'au moins un autre gaz et parfois même plus.

Dans la demande de brevet européen EP-A-323287, la demanderesse a décrit un procédé de conversion thermique du méthane en hydrocarbures de poids moléculaires plus élevés comportant des moyens de chauffage électrique avec transfert de chaleur au mélange gazeux, contenant le méthane à convertir, à travers les parois étanches de gaines en matière céramique qui isolent lesdits moyens de chauffage du mélange gazeux contenant le méthane. Dans ce procédé, la zone de chauffage est chauffée par apport d'énergie électrique à l'aide de résistances électriques et la chaleur dégagée par effet Joule dans ces résistances est transmise, principalement par radiation, aux gaines en matériau céramique disposées autour des résistances de façon non jointive. Les charges gazeuses, qui circulent sensiblement perpendiculairement à l'axe des gaines chauffées, sont chauffées essentiellement par convection et par radiation. Dans la réalisation de ce procédé, on définit au sein du réacteur deux espaces :
- d'une part, l'espace de réaction ou espace process, à l'extérieur des gaines protégeant les résistances, dans lequel circule le mélange de gaz contenant du méthane,
- d'autre part, l'espace des résistances formé par le volume compris entre les résistances proprement dites et les gaines d'isolement dans lequel on introduit de préférence un gaz inerte, c'est-à-dire un gaz exempt de méthane ou de tout hydrocarbure susceptible d'une réaction de conversion thermique ou de tout composé susceptible de réagir violemment avec le méthane ou l'hydrogène. Ce gaz est également choisi de manière à ce qu'il n'endommage pas les résistances utilisées et ne provoque pas un vieillissement accéléré de ces résistances.

L'un des problèmes les plus importants lorsque l'on réalise la conversion thermique du méthane est lié à la formation du coke. En effet, lorsqu'il se forme en quantité trop importante, celui-ci risque d'endommager le four avant les opérations de décokage et par ailleurs, sur le plan économique, sa formation représente une perte importante, aussi bien en ce qui concerne l'énergie électrique consommée que le méthane consommé pour sa formation. Ce problème, bien connu de l'homme du métier, est partiellement résolu par l'introduction dans le mélange de gaz, contenant le méthane à convertir, d'une quantité d'hydrogène représentant de 1 à 90 % en volume par rapport au volume total de gaz. Malgré cette précaution, la formation de coke, principalement au niveau des parois des gaines et sur les autres surfaces de température élevée en contact avec le mélange de gaz contenant le méthane, n'est pas complètement éradiquée.

Ceci explique que, dans la mise en oeuvre de ce procédé de conversion du méthane dans un four de pyrolyse chauffé électriquement, il soit souhaitable :
- d'avoir dans la zone process une quantité d'hydrogène relativement importante,
- de disposer de résistances électriques capables de délivrer à haute température une grande quantité d'énergie par unité de surface et par unité de temps,
- d'avoir des conditions permettant de bons transferts thermiques de manière à ce que la température des éléments chauffants (c'est-à-dire la température de la surface des gaines en contact avec le mélange de gaz contenant du méthane) ne soit pas trop supérieure à la température souhaitée pour effectuer la conversion du méthane.

Dans la mise en oeuvre de ce procédé, il a été précisé qu'il est préférable que l'espace des résistances soit rempli par un fluide gazeux tel que l'azote, le gaz carbonique ou l'air. L'utilisation de l'air ne peut se concevoir que si l'étanchéité assurée par les gaines entre l'espace process et l'espace des résistances est parfaite. En effet, il y aurait autrement un risque important de former un mélange gazeux, à très haute température, comprenant de l'oxygène, du méthane et de l'hydrogène, et donc un risque d'explosion. La réalisation d'un système parfaitement étanche est relativement difficile et de plus nécessite l'utilisation de céramique de très grande étanchéité et donc de très grande qualité, c'est-à-dire une céramique, dont la densité est proche de la densité théorique, exempte de porosité ouverte.

L'emploi d'une telle céramique est d'un coût très élevé, ce qui pénalise le procédé. On est donc de fait conduit à accepter l'utilisation de gaines dont l'étanchéité n'est pas parfaite et à utiliser, soit de l'azote avec le risque, non négligeable, vu la température de peau des résistances, de formation, dans le cas de résistances en carbure de silicium, de nitrure de silicium, ce qui est en principe sans conséquences sur la tenue mécanique des résistances mais provoque une variation de la résistivité de ces résistances et donc accélère leur vieillissement, et ce d'autant plus que la température de la résistance (et donc de l'élément chauffant) est plus élevée et que l'énergie fournie par la résistance est plus importante, soit du dioxyde de canne ou gaz carbonique ce qui, même si le débit de fuite depuis l'espace des résistances vers l'espace process est faible, apporte inévitablement des perturbations au niveau des séparations des produits formés au cours de la conversion thermique du méthane en les compliquant d'une part par sa présence et d'autre part par la présence de monoxyde de carbone et d'eau qui se forme inévitablement par réaction entre le dioxyde de carbone, le méthane, le coke et l'hydrogène dans l'espace process.

Un des objets de l'invention est de remédier aux inconvénients décrits ci-avant. Les objectifs que l'on se propose d'atteindre et qui répondent aux problèmes soulevés par l'art antérieur sont essentiellement les suivants :
- limiter au maximum la formation de coke, en particulier sur les surfaces chaudes telles que par exemple les parois des gaines entourant les résistances,
- utiliser comme gaz dans l'espace des résistances un gaz ou un mélange de gaz comprenant de préférence un gaz déjà présent dans le mélange de gaz circulant dans l'espace process, ce qui permet d'employer des gaines dont l'étanchéité n'a pas besoin d'être très grande,
- améliorer les échanges thermiques entre le mélange gazeux contenant du méthane et les surfaces chaudes en contact avec ce mélange,
- minimiser les problèmes de distribution et de répartition des gaz à l'intérieur du réacteur,
- augmenter la fiabilité du dispositif et sa facilité de construction et de démontage pour le décokage du réacteur et son entretien.

La présente invention propose un procédé et un dispositif pour sa mise en oeuvre apportant des améliorations notables par rapport aux réalisations selon l'art antérieur telles que par exemple une mise en oeuvre plus facile, plus souple et mieux contrôlée et un coût moins élevé aussi bien au niveau des investissements qu'au niveau des utilités.

Plus particulièrement, l'invention concerne un procédé de conversion thermique du méthane en hydrocarbures de poids moléculaires plus élevés dans une zone de réaction, de forme allongée selon une direction, comprenant une zone de chauffage et une zone de refroidissement faisant suite à ladite zone de chauffage et dans laquelle on refroidit les effluents de la zone de chauffage, selon lequel on fait circuler un mélange gazeux renfermant du méthane, dans ladite zone de chauffage, selon une direction d'écoulement sensiblement parallèle à la direction de la zone de réaction, caractérisé en ce que ladite zone de chauffage comporte, entre deux parois en matériau réfractaire, au moins une zone longitudinale, dans laquelle circule ledit mélange gazeux, ladite zone longitudinale comprenant une pluralité d'éléments, disposés en au moins deux nappes, sensiblement parallèles entre elles et sensiblement parallèles à la direction de la zone de réaction, lesdits éléments formant en projection transversale un faisceau à pas triangulaire, carré ou rectangulaire, l'une au moins de ces nappes comprenant une série de gaines à l'intérieur desquelles sont disposés des moyens de chauffage électrique formant ainsi une nappe d'éléments chauffants, lesdits moyens de chauffage étant ainsi isolés du contact direct avec le mélange gazeux renfermant du méthane, et lesdits éléments chauffants étant regroupés par sections successives transversales, comportant chacune au moins une rangée transverse d'éléments, lesdites sections étant sensiblement perpendiculaires à la direction de la zone de réaction, indépendantes entre elles et alimentées en énergie électrique de façon à déterminer au moins deux parties dans la zone de chauffage, la première partie permettant de porter la charge jusqu'à une température au plus égale à environ 1500 °C, et la deuxième partie, subséquente de la première partie, permettant de maintenir la charge à une température sensiblement égale à la température maximum à laquelle elle a été portée dans ladite première partie, et selon lequel on refroidit les effluents de la zone de chauffage par introduction dans la zone de refroidissement d'un fluide de refroidissement, puis on recueille lesdits hydrocarbures de poids moléculaires plus élevés à l'extrémité de la zone réactionnelle.

La zone de chauffage est chauffée par apport d'énergie électrique à travers des moyens de chauffage tels que des résistances électriques ; la chaleur dégagée par effet Joule dans ces résistances est transmise principalement par radiation aux gaines disposées autour des résistances de façon non jointive. Ces gaines sont habituellement en matériau céramique ou en tout matériau réfractaire supportant les températures requises et les atmosphères réductrices et oxydantes du milieu, comme par exemple certains nouveaux alliages métalliques de la firme KANTHAL SA comme le KANTHAL AF ou le KANTHAL APM, ou encore en béton réfractaire. Ces gaines pourront être poreuses ou non. Il est souvent plus facile et moins cher d'utiliser des gaines poreuses laissant passer un mélange gazeux depuis l'espace des résistances vers l'espace process. Le mélange gazeux, contenant du méthane, qui circule dans la zone de chauffage de manière sensiblement perpendiculaire à l'axe des gaines, est chauffé essentiellement par convection et par radiation.

Le couplage thermique déshydrogénant du méthane est une réaction fortement endothermique et nécessite l'obtention, à un niveau élevé de température, de l'ordre de 1100 à 1500 °C, d'une densité de flux thermique très importante. Il est nécessaire que l'apport maximum de chaleur soit effectué dans la zone où s'effectuent les réactions endothermiques de craquage et de déshydrogénation ; par ailleurs, compte tenu de la réactivité des produits formés, comme l'acétylène ou l'éthylène, il est nécessaire d'avoir un temps de contact contrôlé, relativement court, suivi d'une trempe rapide, de façon à obtenir un profil de température de type "carré" et à éviter une trop grande formation de coke.

Les échanges thermiques sont l'un des éléments clé pour ce type de réaction très endothermique où il est nécessaire de transférer des quantités d'énergie très importantes depuis les résistances vers le mélange gazeux contenant du méthane dénommé ci-après gaz process. Au cours de l'étude préliminaire effectué par la demanderesse sur les échanges thermiques dans un four à pyrolyse construit selon le modèle utilisé dans la présente invention, on s'est aperçu que l'échange de chaleur depuis la résistance vers la gaine est un échange essentiellement radiatif, mais en revanche il n'y a pratiquement pas d'échange radiatif entre la gaine et le gaz process. En effet, celui-ci est habituellement essentiellement constitué d'un mélange hydrogène-méthane mélange qui n'absorbe pratiquement pas ou très peu le rayonnement émis par les gaines. Le transfert thermique, entre le gaz process et les gaines, est donc dans le cas envisagé dans la présente invention essentiellement un transfert par convection. Dans un tel cas, la qualité des échanges thermiques sera directement liée à la surface d'échange disponible et au rapport surface/volume.

Ainsi, si la surface d'échange est relativement faible, il sera nécessaire pour obtenir une température de gaz process donnée, correspondant à un taux de conversion préalablement choisi, d'augmenter la température des gaines, dans des proportions d'autant plus importantes que cette surface est faible, ce qui implique un risque accru de formation de coke et également la nécessité d'augmenter la température des résistances, ce qui entraîne un vieillissement plus rapide de ces résistances ou même si le taux de conversion préalablement choisi est très élevé, la quantité d'énergie à transférer devient très grande et le risque de détérioration des résistances augmente très fortement.

Les parois participent de manière importante à l'échange thermique, puisqu'elles sont capables d'absorber le rayonnement émis par les gaines et par conséquent les températures des gaines et des parois ont tendance à s'équilibrer. Il est alors possible d'augmenter notablement la surface d'échange en modifiant la conception du dispositif de la manière suivante : alors que dans la conception initiale, les gaines protégeant les résistances et permettant le transfert de chaleur au gaz process étaient de préférence disposées en quinconce, selon la présente invention elles seront le plus souvent alignées, ce qui permet de constituer n rangées ou nappes de m résistances dans le sens de la longueur (pour un nombre total de résistances égal à n x m), et dans une forme de réalisation préférée d'associer un certain nombre de pseudo éléments chauffants de préférence alignés, ce qui permet de constituer z rangées ou nappes de y pseudo éléments chauffants (pour un nombre total de pseudo éléments chauffants égal à z x y), on formera ainsi au moins une zone longitudinale et le plus souvent au moins deux zones longitudinales comprenant chacune au moins deux nappes d'éléments dont au moins une d'éléments chauffants, chaque zone étant séparée de la suivante par une paroi en matériau réfractaire. Par radiation, la température de ces parois augmente et a tendance à atteindre la même valeur que celle des gaines entourant les résistances. Ces parois participeront donc également au chauffage par convection du gaz process et il en sera de même pour les pseudo éléments chauffants lorsque ceux-ci sont présents. Ainsi, dans cette forme de réalisation, la surface d'échange étant notablement augmentée, on pourra obtenir la même température de gaz process avec une température des gaines et des parois relativement plus faible, ce qui permet en conséquence une diminution de la formation de coke. Dans la présente description, le terme élément chauffant désigne l'ensemble constitué d'une gaine de protection et d'au moins une résistance à l'intérieur de ladite gaine, et le terme pseudo élément chauffant un élément en matériau réfractaire ayant habituellement la même hauteur que l'élément chauffant et dont la section a le plus souvent la même forme et la même surface que celle des éléments chauffants ou une forme similaire ou dérivée et une surface différente. Par exemple, si les éléments chauffants ont une section circulaire, les pseudo éléments chauffants pourront avoir une section circulaire, semi-circulaire ou correspondant à un quart de cercle.

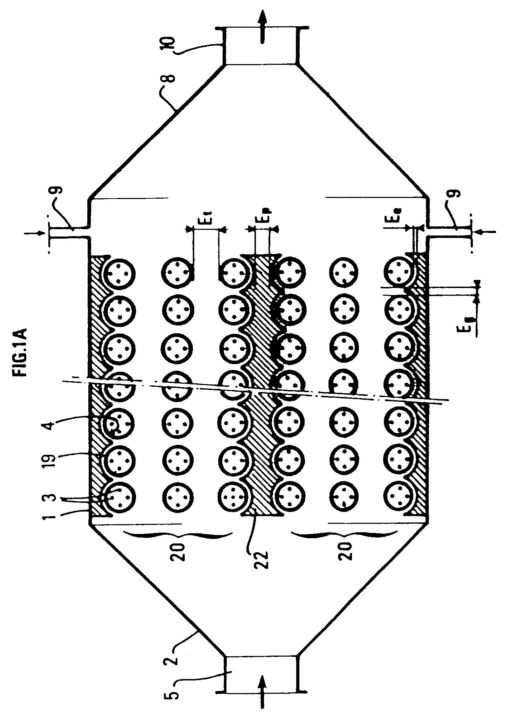

Dans cette forme de réalisation, les échanges convectifs entre le gaz process et les parois sont largement augmentés et ils peuvent être encore améliorés en imposant au gaz des vitesses importantes et en créant des zones de turbulence. L'augmentation de la vitesse des gaz peut par exemple être obtenue en utilisant des parois dont la forme favorise cette augmentation de vitesse et l'apparition de zones de turbulence. Des parois de formes particulières sont représentées à titre non limitatif sur la figure 1A. Les parois et les pseudo éléments chauffants sont habituellement en matériau réfractaire. Tout matériau réfractaire peut être utilisé pour réaliser les parois et ces pseudo éléments, et on peut citer à titre d'exemples non limitatifs la zircone, le carbure de silicium, la mullite et divers bétons réfractaires. Dans une forme préférée de réalisation, les éléments chauffants comporteront une gaine en matériau céramique.

Etant donné qu'il n'est nullement indispensable d'avoir une étanchéité au niveau des parois, puisque la composition du gaz est pratiquement identique de chaque côté des parois, cette réalisation n'induit qu'une augmentation minime du coût du four. En effet, d'une part il n'est pas nécessaire d'avoir des parois spécialement épaisses, ni une réalisation particulièrement complexe, d'autre part les dimensions globales du four augmentent peu car l'essentiel de la largeur de ce four est due à la largeur des gaines. A titre d'exemple, les gaines peuvent avoir une largeur de l'ordre de 150 mm, pour une épaisseur de paroi ayant une largeur de l'ordre de 50 mm, ce qui ne provoque qu'une augmentation de largeur globale du four inférieure à environ 30 % . Par ailleurs, il est préférable que chaque paroi comporte au moins un moyen permettant d'équilibrer les pressions dans les zones longitudinales situées de part et d'autre de la paroi. A titre d'exemple de moyen simple mais efficace permettant d'équilibrer les pressions, on peut citer la création de zones comportant une ou plusieurs perforations ou de zones poreuses.

Un avantage supplémentaire de cette réalisation comportant des parois est de permettre une réalisation plus simple du four, les parois verticales permettant, outre l'amélioration du transfert thermique par convection, de supporter la voûte du four. A noter que selon la forme préférée de réalisation de l'invention, les pseudo éléments chauffants améliorent également le transfert thermique et participent également au maintien de la voûte du four.

Selon l'une des caractéristiques de l'invention, les résistances électriques qui fournissent de la chaleur à la zone de chauffage sont alimentées de façon indépendante en énergie électrique soit isolément, soit par rangées transverses, soit encore par petits groupes, de façon à définir des sections de chauffage le long de la zone de chauffage et à pouvoir ainsi moduler la quantité d'énergie fournie tout au long de cette zone. La zone de chauffage est habituellement composée de 2 à 20 sections de chauffage, de préférence de 5 à 12 sections. Dans la première partie de cette zone, le mélange gazeux renfermant du méthane, préalablement chauffé à environ 750 °C, est habituellement porté à une température au plus égale à environ 1500 °C, et avantageusement entre 1000 et 1300 °C (le début de la zone de chauffage est situé à l'endroit où la charge est introduite).

La modulation de ces sections de chauffage est réalisée de façon classique ; les éléments chauffants correspondant aux sections précitées sont en général alimentés par des ensembles modulateurs à thyristors. Des transformateurs permettent éventuellement d'adapter les tensions à priori, alors que les modulateurs permettent le réglage fin et continu de la puissance injectée.

Afin de permettre la régulation de l'ensemble, chaque section de chauffage peut être munie d'une canne pyrométrique à thermocouple adaptée au niveau de température : ces cannes sont disposées dans les espaces où circule la charge, les informations sont transmises au régulateur qui commande le modulateur à thyristor.

Dans la première partie de la zone de chauffage, l'énergie électrique sert presque exclusivement à porter le mélange réactionnel de sa température initiale (par exemple environ 750 °C) à la température où les réactions endothermiques de couplage déshydrogénant du méthane ont lieu (par exemple environ 1200 °C). C'est donc au début de la deuxième partie de la zone de chauffage qu'il est nécessaire de fournir l'énergie maximum au milieu réactionnel, ce qui est facilement réalisé par exemple par la modulation d'une ou plusieurs sections de chauffage et/ou par l'utilisation de modules (décrits ci-après) pour la fabrication du four, comportant un nombre de pseudo éléments différent suivant qu'ils se situent au début de la première partie de la zone de chauffage ou vers le milieu ou la fin de cette zone et de même suivant leurs positions dans la deuxième partie de la zone de chauffage.

La longueur de la première partie de la zone de chauffage représente habituellement de 20 à 80 % de la longueur totale de la zone de chauffage, avantageusement de 30 à 70 % . L'énergie électrique fournie à cette première partie de la zone de chauffage est telle qu'elle génère un fort gradient de température, habituellement d'environ 0,5 à environ 25 °C/cm, et avantageusement d'environ 1 à environ 20 °C/cm.

Dans la deuxième partie de la zone de chauffage, on module l'énergie électrique fournie aux différentes sections de chauffage de cette zone de façon à ce que la variation de température tout au long de cette zone soit faible, habituellement inférieure à environ 50 °C (+ ou - 25 °C autour de la valeur de la consigne) et avantageusement inférieure à environ 20 °C. Par ailleurs, l'utilisation de différentes sections transversales de chauffage (ainsi que, par exemple, celle de différents modules comportant un nombre plus ou moins grand de pseudo éléments chauffants) indépendantes les unes des autres permet d'apporter, au niveau de la deuxième partie de la zone de chauffage, le maximum d'énergie thermique à l'endroit où s'effectue la plus grande partie des réactions endothermiques, et de maintenir dans le reste de la zone de chauffage une température quasi uniforme.

La longueur de la zone de chauffage est habituellement d'environ 50 à environ 90 % de la longueur totale de la zone réactionnelle.

On obtient, notamment dans les conditions de chauffage ci-dessus, un flux thermique très important à un niveau de température élevé. Ceci implique habituellement un choix particulier pour le matériau constitutif des résistances qui doit, outre le fait d'être résistant à l'atmosphère dans laquelle baignent les résistances dans les conditions de température du fonctionnement, être capable de délivrer une puissance par unité de surface relativement importante. A titre d'exemple de matériau utilisable pour la réalisation des résistances, on peut citer le carbure de silicium, le nitrure de bore, le nitrure de silicium et le bisiliciure de molybdène (MoSi₂). On préfère habituellement employer des résistances en bisiliciure de molybdène qui présentent de nombreux avantages lors de leur utilisation à haute température :
- elles acceptent une charge (puissance émise par unité de surface) importante qui peut aller jusqu'à 20 W/cm²,
- elles peuvent travailler à très haute température,
- elles accusent dans le temps un vieillissement négligeable,
- elles supportent facilement des atmosphères réductrices à des températures élevées. Dans le procédé de l'invention, la zone de chauffage est suivie d'une zone de refroidissement (ou trempe) de façon à abaisser très rapidement la température des effluents de la zone de chauffage vers environ 300 °C par exemple.

Selon un mode de réalisation, on procède à une trempe directe : les effluents de réaction quittent la zone de chauffage et sont très rapidement refroidis par une mise en contact direct avec un fluide de refroidissement qui est injecté dans les effluents au moyen d'au moins un injecteur, habituellement en matériau céramique, disposé à la périphérie du réacteur. On peut utiliser, comme fluide de refroidissement, des gaz de pétrole liquéfiés, du propane, des huiles hydrocarbonées ou de l'eau. Le propane est le gaz: de trempe préféré car il peut également être partiellement craqué et contribuer ainsi à la formation de produits tels que l'éthylène. Les effluents totaux résultant du mélange sont ensuite recueillis et séparés.

Selon un mode de réalisation préféré, les effluents de réaction issus de la zone de chauffage sont refroidis par mise en contact indirect avec un fluide de refroidissement, par exemple en faisant circuler ledit fluide dans des conduits étanches à l'intérieur de la zone de refroidissement. L'ensemble de ces caractéristiques permet d'obtenir, grâce à ce procédé, une conversion thermique du méthane en acétylène, éthylène et produits benzéniques s'effectuant avec un bon taux de conversion et une sélectivité en ces produits élevée.

Les charges hydrocarbonées utilisables dans le cadre de l'invention sont des charges gazeuses dans les conditions normales de température et de pression, comprenant habituellement un pourcentage en volume de méthane d'au moins 10 %, par exemple de 10 à 99 %, le plus souvent de 20 à 99 % et préférentiellement de 30 à 80 %. Le reste de la charge comprend, comme précisé ci-avant, dans presque tous les cas une proportion en volume d'hydrogène allant le plus souvent de 1 à 90 %. Elle peut également comprendre d'autres gaz, tels que, par exemple, des hydrocarbures aliphatiques, saturés ou non, comprenant un nombre d'atomes égal ou supérieur à 2 comme l'éthylène, l'éthane, le propane ou le propylène. Elle peut aussi comprendre de l'azote, du dioxyde de carbone ou du monoxyde de carbone.

On peut, en restant dans le cadre de l'invention, rajouter aux charges définies ci-avant de la vapeur d'eau de dilution ; le rapport pondéral de la vapeur d'eau de dilution à la charge hydrocarbonée est en général de l'ordre de 0,1 : 1 à 1 : 1.

Les charges à traiter ont un temps de séjour dans la zone réactionnelle habituellement d'environ 2 millisecondes à environ 1 seconde et de préférence d'environ 30 à environ 400 millisecondes. Comme gaz dit d'étanchéité, c'est-à-dire comme gaz introduit dans l'espace des résistances (cet espace est celui dont la définition a été donnée ci-avant en liaison avec l'analyse de la demande de brevet européen EP-A-323287), on choisira de préférence un gaz qui permettra d'obtenir la meilleure longévité des résistances tout en apportant, par suite du débit de fuite dudit gaz vers l'espace process, un minimum de complications tant au niveau de la réaction elle-même (formation de coke minimum) qu'au niveau de la séparation des produits en aval du réacteur parfois dénommé four de pyrolyse. Ainsi on pourra choisir comme gaz d'étanchéité de l'azote, du gaz carbonique, de l'hydrogène ou un mélange de deux ou plusieurs de ces gaz. On peut également utiliser comme gaz d'étanchéité l'un des gaz mentionnés ci-avant renfermant de la vapeur d'eau. On préfère habituellement introduire dans les gaines entourant les résistances un gaz contenant de l'hydrogène. Ce gaz peut être de l'hydrogène sensiblement pur, de l'hydrogène industriel ou un mélange d'hydrogène avec un autre gaz inerte tel que par exemple de l'azote, de l'hélium, de l'argon, de la vapeur d'eau ou du gaz carbonique. On utilise de préférence de l'hydrogène pur ou industriel ou un mélange d'hélium et d'hydrogène ou un mélange d'argon et d'hydrogène ou un mélange de vapeur d'eau et d'hydrogène contenant habituellement au moins 5 % en volume d'hydrogène et de préférence au moins 10 % . Lorsqu'on utilise un mélange d'azote et d'hydrogène, celui-ci contient habituellement au moins 25 % en volume d'hydrogène et de préférence au moins 50 %.

Dans une forme préférée de réalisation, les moyens de chauffage électrique seront isolés du contact direct avec le mélange gazeux renfermant du méthane par des gaines dans lesquelles on introduit un gaz contenant de l'hydrogène, lesdites gaines ayant une perméabilité appropriée et ledit gaz étant introduit à l'intérieur desdites gaines à une pression telle qu'il y a diffusion, au moins en certains points, d'au moins une partie de l'hydrogène, contenu dans le gaz introduit dans l'espace des résistances, vers l'espace process, c'est-à-dire depuis l'intérieur desdites gaines vers l'extérieur desdites gaines, cet hydrogène pouvant se diluer alors dans le mélange gazeux renfermant du méthane.

On ne sortirait pas du cadre de l'invention dans le cas où la perméabilité des gaines serait telle qu'elle permette la diffusion de l'ensemble des composés gazeux, contenu dans le gaz introduit dans l'espace des résistances, vers l'espace process. Cette perméabilité peut résulter d'une étanchéité sur chaque gaine, réalisée volontairement de façon imparfaite et/ou de l'utilisation d'un matériau constitutif des gaines ayant une porosité ouverte permettant le passage de l'hydrogène, c'est-à-dire en d'autres termes un matériau perméable à l'hydrogène. Le plus souvent, il est recommandé d'utiliser un matériau perméable.

Ainsi, selon une réalisation préférée de l'invention, les gaines, isolant les moyens de chauffage électrique du contact direct avec le mélange gazeux renfermant du méthane, sont constituées d'un matériau poreux dont la porosité est suffisante pour permettre la diffusion de l'hydrogène à travers lesdites gaines. Ces gaines sont ainsi, de préférence, fabriquées en matière céramique poreuse présentant une porosité ouverte d'au moins environ 1 % et d'au plus environ 40 % en volume par rapport au volume de la paroi de la gaine, et habituellement d'environ 5 % à environ 30 % .

L'utilisation de l'hydrogène sensiblement pur qui diffuse, au moins en partie vers l'espace process, procure plusieurs avantages. Il ne complique pas les séparations en aval du four à pyrolyse puisque le gaz à craquer est habituellement un mélange de méthane ou gaz naturel et d'hydrogène dans une proportion en volume de préférence de 10 à 80 % d'hydrogène et le plus souvent de 30 à 70 %.

L'introduction d'hydrogène tout le long du four de pyrolyse permet de diminuer la taille globale du four. En effet, si l'on vise en sortie une certaine proportion d'hydrogène dans le gaz craqué, en entrée du four celle-ci sera moindre et pour un même temps de séjour à respecter, le volume réactionnel sera moindre, donc la taille du four également. Mais, de plus, cette réalisation amènera une proportion croissante d'hydrogène le long du four de pyrolyse, ce qui représente un avantage du point de vue de la cinétique de craquage et de la stabilité des produits, car en début de four, une trop grande quantité d'hydrogène inhiberait par trop les réactions de craquage, mais en fin de four, alors qu'il y a une quantité notable de produits formés, notamment d'éthylène et d'acétylène, il est avantageux d'avoir une quantité d'hydrogène plus importante afin d'éviter la formation de coke. De l'hydrogène pénétrant dans la zone process au niveau de la réalisation de chaque étanchéité (au moins une par gaine) sur chaque gaine protégeant les résistances, et/ou à travers la paroi des gaines par diffusion, on aura donc bien l'effet désiré.

Par ailleurs, selon une forme préférée de réalisation du procédé de la présente invention, étant donné qu'il n'est pas indispensable de rechercher des étanchéités aussi parfaites que possible entre l'espace process et l'espace des résistances, on diminue le coût de réalisation du four tout en diminuant également les contraintes thermo-mécaniques au niveau des raccords des gaines, ce qui augmente la fiabilité de l'ensemble du dispositif. Un autre avantage réside dans le choix qu'il est alors possible de faire quant aux gaines de protection des résistances, délimitant l'espace process de l'espace des résistances. En effet, lorsque l'on utilise comme gaz d'étanchéité de l'azote ou du CO₂, il est nécessaire pour de multiples raisons de limiter la consommation de ce gaz, c'est-à-dire la fuite dudit gaz hors de l'espace des résistances vers l'espace process. Cela est habituellement réalisé par la recherche d'une étanchéité aussi parfaite que possible, au niveau notamment des jonctions gaines-reste du four. Cela est aussi réalisé par l'utilisation de gaines en céramiques, et en particulier en carbure de silicium aussi étanches que possibles, c'est-à-dire de très grande qualité et donc de très grand prix.

Il est en effet bien connu de l'homme du métier qu'il existe de nombreuses variétés de céramique, et notamment de carbure de silicium, qui proviennent de qualité de poudre constitutive et de conditions de frittage très différentes. Sans vouloir entrer dans le détail, on peut cependant noter qu'un des critères de qualité est lié à la plus faible rémanence de porosité après frittage. Il est bien connu que si une partie de cette porosité se trouve être fermée, c'est-à-dire sans effet sur l'étanchéité globale du matériau, une autre partie, non négligeable, surtout pour le carbure de silicium le plus courant, est une porosité ouverte et que notamment il y a, à haute température, diffusion de l'hydrogène à travers ce matériau. On comprend donc bien que lorsque l'on utilise comme gaz d'étanchéité un gaz tel que l'azote ou le CO₂, on doit utiliser un carbure de silicium de très haute qualité, dont la densité est proche de la densité théorique, donc exempt quasiment de porosité ouverte, pour éviter d'une part une fuite dudit gaz de la zone des résistances vers l'espace process, et d'autre part la différence de pression partielle de l'hydrogène étant positive dans le sens process-résistances, une diffusion de l'hydrogène contenu dans le gaz process vers l'espace des résistances.

L'utilisation de gaines en céramique, notamment en carbure de silicium, de qualité moyenne, comportant une porosité ouverte d'au moins environ 1 % en volume (par exemple d'environ 20 % en volume) est ainsi non seulement possible mais même souhaitable, ce qui abaisse le coût de réalisation du four. Par ailleurs, l'existence même de cette porosité ouverte crée en surface de la gaine en céramique côté espace process une pression partielle d'hydrogène isolant en quelque sorte la surface de la céramique du gaz process ce qui, sans vouloir être lié par une quelconque théorie, explique la réduction notable de la formation de coke puisque celui-ci se forme habituellement, pour l'essentiel, à la surface des gaines et qu'au contraire les produits formés se trouveront dans une atmosphère locale plus riche en hydrogène, donc moins favorable à la formation de coke.

Par porosité ouverte, on désigne dans la description de la présente invention la porosité constituée de microcavités incluses dans les pièces en céramiques massives considérées, l'adjectif ouvert signifiant qu'il y a libre passage d'une part entre la plupart desdites microcavités, et d'autre part entre lesdites microcavités et les surfaces internes et externes des pièces considérées ; la notion de libre passage doit aussi être considérée en fonction de la nature du milieu et des conditions physiques dans lesquels se trouve la céramique. En particulier, pour des molécules de petites tailles comme l'hydrogène ou l'hélium, le libre passage sera facile, d'autant plus d'ailleurs s'il l'on a une différence de pression entre les deux surfaces de la pièce en céramique. Dans ce cas, la pièce est dite perméable, à l'hydrogène par exemple, ou non étanche. Par porosité fermée, on désigne dans la description de la présente invention la porosité constituée de microcavités ne communiquant pas avec la surface de la pièce. Dans ce cas là, cette porosité fermée ne provoque qu'une diminution globale de la densité de la pièce.

L'invention a également pour objet le dispositif pour la mise en oeuvre du procédé. Ce dispositif peut également être utilisé pour la réalisation d'autres réactions endothermiques s'effectuant habituellement à des températures supérieures à environ 600 °C, et par exemple d'environ 700 à environ 1450 °C, avec des temps de séjour de l'ordre de 2 millisecondes à quelques secondes, par exemple jusqu'à 20 secondes.

Plus particulièrement, l'invention concerne un dispositif pour la mise en oeuvre du procédé comprenant un réacteur (1), de forme allongée, selon un axe de préférence à section carrée ou rectangulaire, comportant à une première extrémité des moyens d'alimentation en mélange gazeux, à l'extrémité opposée des moyens d'évacuation des effluents produits et entre ces deux extrémités des moyens d'alimentation en fluide de refroidissement, caractérisé en ce que ledit réacteur comprend dans une première partie (côté première extrémité) au moins une zone longitudinale entre deux parois en matériau réfractaire sensiblement parallèles entre elles et sensiblement parallèles à l'axe du réacteur, ladite zone longitudinale comprenant une pluralité d'éléments, disposés en au moins deux nappes, sensiblement parallèles entre elles et sensiblement parallèles à l'axe du réacteur, l'une au moins de ces nappes comprenant une série de gaines (4) à l'intérieur desquelles sont disposés des moyens de chauffage électrique (3) formant ainsi une nappe d'éléments chauffants, lesdits éléments étant disposés de façon à définir entre eux et/ou entre les nappes qu'ils forment et/ou entre eux et les parois des espaces ou passages pour la circulation des mélanges gazeux et/ou des effluents, lesdits moyens de chauffage et les dites gaines étant adaptés à chauffer lesdits passages par section transversales successives, indépendantes et sensiblement perpendiculaires à l'axe du réacteur, chaque section transversale comportant au moins une rangée transverse d'éléments et ledit réacteur comportant en outre des moyens d'asservissement et de modulation de chauffage reliés auxdits moyens de chauffage, et comportant dans une deuxième partie (8) (côté extrémité opposée) contiguë à la première partie des moyens de refroidissement (9) des effluents reliés auxdits moyens d'alimentation en fluide de refroidissement.

Selon une forme préférée de réalisation, ledit dispositif comprendra des moyens d'introduction, à une pression appropriée, d'un gaz contenant de l'hydrogène à l'intérieur des gaines (4) et lesdites gaines seront des gaines ayant une perméabilité suffisante pour permettre, au moins en certains points, la diffusion d'au moins une partie de l'hydrogène depuis l'intérieur desdites gaines vers l'extérieur desdites gaines, cet hydrogène se diluant alors dans ledit mélange gazeux.

Les moyens d'introduction, à une pression appropriée, du gaz sont ceux connus de l'homme du métier. Ils peuvent comprendre par ailleurs des moyens de régulation et de contrôle des pressions régnant à l'intérieur et à l'extérieur desdites gaines. Lesdits moyens de refroidissement sont des moyens adaptés à refroidir par contact direct ou par contact indirect les effluents quittant la première zone.

Les gaines entourant les résistances, habituellement de façon non jointive, peuvent être disposées de façon superposée ou en quinconce et peuvent former en projection transversale un faisceau à pas triangulaire, carré ou rectangulaire.

Le nombre total de nappes comportant des moyens de chauffage et le nombre des moyens de chauffage dans chaque gaine et par nappe ne sont pas déterminants dans le procédé ; ils sont évidemment fonction de la dimension des moyens de chauffage, des gaines qui les entourent et des parois séparant les zones longitudinales. Les éléments chauffants peuvent être identiques entre eux ou différents, tant par leurs dimensions que par leur puissance de chauffage. A titre d'exemple, un élément chauffant pourra comprendre à l'intérieur de la gaine de 1 à 5 et le plus souvent de 1 à 3 résistances.

Le nombre d'éléments chauffants détermine la puissance électrique maximum disponible pour un volume réactionnel donné, et influe également sur le temps de séjour de la charge ; il sera choisi en fonction du débit de charge admissible, compte tenu de ces paramètres.

On peut, dans le cadre de la présente invention, réaliser l'ensemble du réacteur, zone de chauffage et zone de trempe, soit sous forme monobloc, soit encore par juxtaposition, jointive ou non, de divers modules, le plus souvent de formes identiques, qui sont assemblés entre eux par tout moyen utilisable, par exemple à l'aide de brides. Ainsi le réacteur pourra par exemple comporter au moins deux zones longitudinales formées par la juxtaposition dans le sens longitudinal et dans le sens transversal d'une série de modules comportant chacun au moins une paroi longitudinale en matériau réfractaire. Dans une forme préférée de réalisation, chaque paroi en matériau réfractaire séparant deux zones longitudinales adjacentes comporte au moins un moyen permettant l'équilibrage des pressions entre les deux zones. Lorsque le réacteur est formé par l'association de modules, ceux-ci seront de préférence assemblés de manière non étanche, de sorte que des gaz puissent passer au niveau de l'assemblage d'une zone longitudinale à la zone longitudinale située de l'autre côté de la paroi. Le réacteur comporte habituellement de 1 à 20 et de préférence de 2 à 8 zones longitudinales. L'un des avantages de réaliser le four par association de modules consécutifs réside dans l'unité qui en découle. On pourra par exemple associer à chaque module un éléments de voûte du four, ainsi qu'une régulation de chauffage et de puissance, le module constituant alors une section de chauffage. Par ailleurs, le démontage et l'entretien du four s'en trouveront simplifiés. Selon une autre forme de réalisation, plusieurs modules pourront être associés pour former une section de chauffage. Il est également possible d'installer les divers modules dans le four comportant des parois, continues ou non, qui ont été préalablement positionnées. Les moyens de chauffage électrique utilisables dans le cadre de la présente invention sont de préférence des résistances chauffantes dont le matériau constitutif doit être résistant à l'atmosphère dans laquelle elles baignent. Dans une forme préférée de réalisation, on utilise des résistances fabriquées en un matériau supportant une atmosphère globalement réductrice, jusqu'à des températures de l'ordre de 1500 °C ; on préfère utiliser des résistances en bisiliciure de molybdène, par exemple des résistances en épingle.

Les éléments présents dans chaque zone longitudinale sont de préférence des éléments sensiblement cylindriques ou tubulaires ayant tous un diamètre externe sensiblement égal et une hauteur sensiblement égale, et dans lequel ceux renfermant les moyens de chauffage et formant une nappe d'éléments chauffants sont des gaines cylindriques ou tubulaires dont le diamètre interne D est d'environ 1,2 à environ 8 fois, et le plus souvent d'environ 1,5 à environ 4 fois le diamètre maximal d du cercle englobant lesdits moyens de chauffage, et les autres éléments sont des éléments creux tels que des gaines cylindriques ou tubulaires ou des éléments cylindriques pleins.

Ces gaines en matériau réfractaire sont le plus souvent en céramique. On peut utiliser des céramiques comme la mullite, la cordiérite, le nitrure de silicium, le carbure de silicium, la silice ou l'alumine ; le carbure de silicium est le matériau préférentiellement choisi car il présente une bonne conductivité thermique. Les zones longitudinales sont séparées par des parois fabriquées en un matériau qui peut être le même que celui utilisé pour fabriquer les gaines, mais qui est souvent différent, en particulier pour des questions de coût de fabrication du four. Dans une forme préférée de réalisation du réacteur, chaque zone longitudinale est formée par la juxtaposition d'une série de modules constitutifs, de section carrée ou rectangulaire, comprenant chacun au moins deux éléments formant une rangée transverse, dont l'un au moins est formé d'une gaine (4), à l'intérieur de laquelle est disposé un moyen de chauffage électrique (3), et constitue un élément chauffant (19), lesdits éléments étant disposés de façon à définir, entre eux et/ou entre eux et les parois de ladite zone, des espaces ou passages pour la circulation des mélanges gazeux et/ou des effluents, et lesdits modules étant juxtaposés de manière à ce que les éléments forment, entre deux parois en matériau réfractaire sensiblement parallèles entre elles et sensiblement parallèles à l'axe du réacteur, au moins deux nappes, sensiblement parallèles entre elles et sensiblement parallèles à l'axe du réacteur.

Chaque module constitutif pourra comporter au moins deux rangées transverses de deux ou trois éléments chauffants disposés de manière à ce que la juxtaposition de ces modules permette d'obtenir au moins deux nappes d'éléments chauffants, lesdites nappes étant perpendiculaires à l'axe du réacteur et lesdits éléments formant en projection transversale un faisceau à pas triangulaire, carré ou rectangulaire.

Selon une autre forme de réalisation, chaque module constitutif pourra comporter au moins deux rangées transverses dont l'une au moins est formée d'éléments chauffants et dont au moins une autre contigué à une rangée d'éléments chauffants est formée de pseudo éléments chauffants en matériau réfractaire, lesdits éléments étant disposés de manière à ce que la juxtaposition de ces modules permette d'obtenir au moins deux nappes d'éléments, lesdites nappes étant perpendiculaires à l'axe du réacteur et lesdits éléments formant en projection transversale un faisceau à pas triangulaire, carré ou rectangulaire. Selon cette dernière forme de réalisation, chaque module constitutif pourra comporter des pseudo éléments chauffants sur sa périphérie, lesdits pseudo éléments chauffants ayant une section telle que par juxtaposition des modules, ceux situés au niveau de l'arête par laquelle lesdits modules sont juxtaposés forment des pseudo éléments ayant sensiblement la même section que les éléments chauffants, ceux contigus à un côté ont une section inférieure à la section des éléments chauffants et de préférence une section égale à environ la moitié de la section desdits éléments chauffants et égale à environ le quart pour ceux contigus à deux côtés du module.

Les éléments sont disposés en nappes parallèles sensiblement perpendiculaires au sens d'écoulement de la charge (gaz process), préférentiellement sensiblement alignés, de façon à ce que la distance qui sépare deux éléments voisins soit la plus réduite possible, tout en tenant compte des impératifs de perte de charge admissible ; la distance entre les éléments de deux nappes voisines ou celle entre les éléments d'une nappe et la paroi la plus proche est habituellement la même que celle entre deux éléments consécutifs dans une nappe donnée.

Cette distance sera habituellement telle que les passages formés entre les éléments ou entre les éléments et la paroi plus proche, passages dans lesquels circule le mélange gazeux renfermant du méthane, auront une dimension d'environ 1 à environ 100 mm, et le plus souvent d'environ 5 à environ 40 mm.

Selon un mode particulier de réalisation de l'invention, les espaces libres ou passages définis ci-avant, destinés à la circulation du gaz process, sont au moins partiellement occupés par des garnissages, habituellement en céramique, préférentiellement conducteurs de la chaleur. On peut ainsi, pour un type de réacteur donné, diminuer le temps de séjour de la charge dans ce réacteur tout en homogénéisant l'écoulement du mélange gazeux et en répartissant mieux la chaleur dissipée. Ces garnissages peuvent avoir des formes diverses, et se présenter par exemple sous forme d'anneaux (anneaux de Raschig, de Lessing ou de Pall), de selles (selles de Berl), de barreaux, de tubes cylindriques fermés. L'invention sera mieux comprise par la description de quelques modes de réalisation, donnés à titre purement illustratif mais nullement limitatif, qui en sera faite ci-après à l'aide des figures annexées, sur lesquelles les organes similaires sont désignés par les mêmes chiffres et lettres de référence.
- La figure 1A représente une coupe longitudinale d'un réacteur suivant un plan perpendiculaire à l'axe des éléments. Dans le cas de la figure 1A, ce réacteur ne comporte dans la zone de chauffage que des éléments chauffants.
- Les figures 1B et 1C représentent une coupe longitudinale d'un réacteur suivant l'axe des éléments.
- La figure 2 illustre un détail de réalisation de la zone de chauffage dans un plan identique à celui des figures 1B et 1C.
- Les figures 3A, 3B, 3C et 4A, 4B, 4C montrent une coupe longitudinale de divers modules de construction du four.

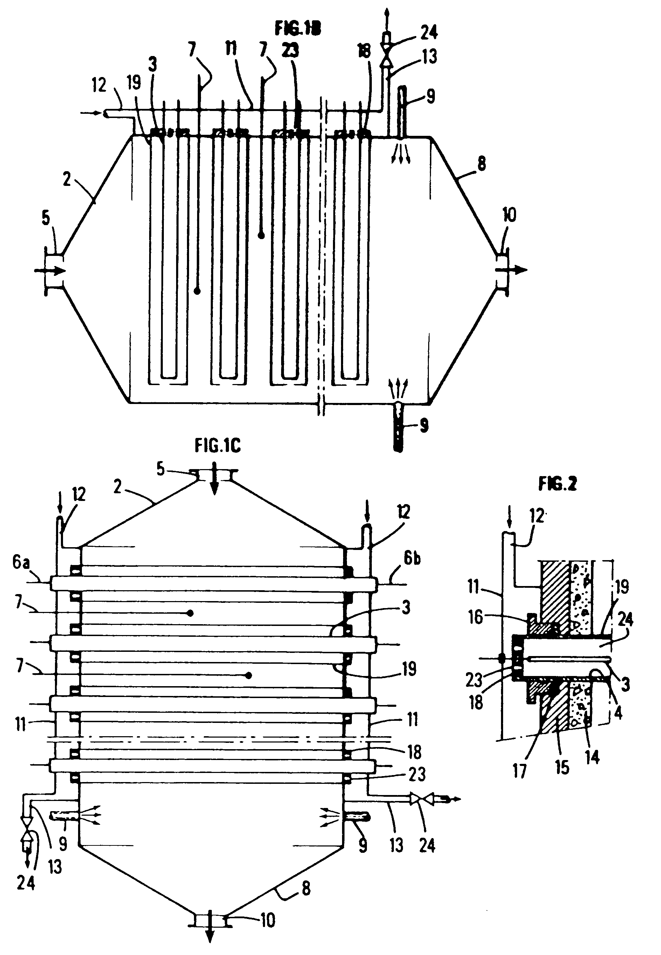

Sur la figure 1A, on a représenté, selon un mode de réalisation, un réacteur (1) sensiblement horizontal de forme allongée et de section rectangulaire, comprenant un distributeur (2) permettant d'alimenter, par un orifice d'entrée (5), le réacteur en mélange gazeux réactionnel. Ce dernier, qui contient par exemple 50 % de méthane, a été préchauffé dans une zone de préchauffage conventionnelle, non représentée sur la figure, de préférence par convection. Le réacteur comprend deux zones longitudinales (20) séparées par une paroi (22), avantageusement en matière céramique, comportant chacune une pluralité d'éléments chauffants (19), comprenant des moyens de chauffage électrique (3) entourés de gaines (4), disposés en nappes parallèles et formant dans un plan (plan de la figure) un faisceau à pas carré. Ces nappes définissent des sections de chauffage transversales sensiblement perpendiculaires à l'axe du réacteur défini selon la direction d'écoulement de la charge. Cette paroi a une forme, adaptée à créer des turbulences, comportant des alvéoles au niveau de chaque élément (19). Ce réacteur comporte également sur ses côtés, parallèles à la direction d'écoulement de la charge, des parois ayant une forme adaptée à créer des turbulences, comportant des alvéoles au niveau de chaque élément (19). Les sections de chauffage sont alimentées en énergie électrique, de façon indépendante, grâce à une paire d'électrodes (6a, 6b sur les figures 1B et 1C), des sondes pyrométriques à thermocouple (7 sur les figures 1B et 1C) sont logées dans les espaces où circule la charge entre les éléments (19) et permettent de réguler automatiquement la température de chaque section de chauffage, par un dispositif classique de régulateur et de modulateur non représenté sur les figures.

Dans la première partie de la zone de chauffage, les éléments sont chauffés de façon à ce que la température de la charge passe rapidement de 750 °C (température de préchauffage) à 1200 °C environ ; cette zone de chauffage progressif représente en général environ 65 % de la longueur totale de la zone de chauffage ; le mélange gazeux circule ensuite dans la deuxième partie de la zone de chauffage où l'on maintient généralement la température à une valeur constante sensiblement égale à celle atteinte à la fin de la première zone de chauffage, soit en général 1200 °C environ. A cet effet, on module la puissance électrique fournie à plusieurs sections de chauffage qui constituent la deuxième partie de la zone de chauffage ; on parvient ainsi à obtenir une variation de température ne dépassant pas environ 10 °C autour de la valeur de la consigne. La longueur de cette deuxième zone de chauffage représente environ 35 % de la longueur totale de la zone de chauffage.

A la sortie de la zone de chauffage, les effluents de la réaction sont refroidis dans une zone de refroidissement (8). Ils sont mis en contact avec un agent de trempe tel que du propane introduit par l'intermédiaire d'injecteurs (9), de trempe, disposés à la périphérie du réacteur (1) et reliés à une source extérieure de propane non représentée. L'ensemble des gaz effluents est refroidi à une température d'environ 500 °C et recueilli par un orifice de sortie 10 à l'extrémité de la zone réactionnelle (1).
Selon un autre mode non illustré, les effluents peuvent être refroidis en circulant à travers des conduits étanches disposés dans la zone (8) par lesquels s'écoule l'agent de trempe, ces conduits étant reliés à la source extérieure de l'agent de trempe.

La figure 1B représente, pour un réacteur horizontal, les mêmes éléments que ceux décrits en liaison avec la figure 1A ; on a représenté, de plus, un boîtier de protection (11) comportant un orifice (12) par lequel on introduit le gaz contenant de l'hydrogène et un orifice (13) muni d'une vanne (24) permettant de réguler le flux du gaz contenant de l'hydrogène. Ce boîtier (11) est fixé sur l'armature métallique du réacteur (1) et entoure les éléments chauffants, formés par l'ensemble des résistances électriques et des gaines les contenant, à l'exception de l'extrémité des résistances électriques par où se fait l'alimentation en énergie électrique. Les résistances (3), en épingle, sont positionnées dans les gaines (4) à l'aide de rondelles (18), par exemple en fibre céramique, comportant des passages (23) permettant au gaz contenant de l'hydrogène de pénétrer dans l'espace compris entre les résistances et les gaines.

Selon une forme de réalisation recommandée, le réacteur comprendra un boîtier (11) cloisonné de sorte que l'on définit des zones transverses qui sont chacune alimentée séparément en gaz d'étanchéité. Cette forme de réalisation permet de limiter l'influence de la perte de charge du réacteur sur le débit de fuite du gaz d'étanchéité depuis l'espace des résistance vers l'espace process et permet donc un meilleur contrôle de ce débit de fuite. La figure 1C représente, pour un réacteur vertical, les mêmes éléments que ceux décrits en liaison avec la figure 1A ; on a représenté, de plus, les boîtiers de protection (11) munis d'orifice (12) et (13) permettant la circulation dans les boîtiers du gaz contenant l'hydrogène qui pénètre dans l'espace des résistances par les orifices (23) des rondelles (18) assurant le positionnement des résistances. Les orifices (13) sont munis de vannes (24) permettant une régulation plus facile du flux du gaz contenant de l'hydrogène. La circulation du gaz contenant de l'hydrogène est habituellement effectuée en légère surpression par rapport à la pression du gaz process au sein du réacteur, assurant ainsi une atmosphère parfaitement contrôlée et une meilleure diffusion de l'hydrogène contenu dans ce gaz vers l'espace process.

La pression pourrait être pratiquement égale à celle du gaz: process et, dans ce cas comme dans le cas d'une surpression globale, il est habituellement préférable que la pression partielle de l'hydrogène soit légèrement plus élevée dans l'espace des résistances que dans l'espace process de manière à être certain que l'hydrogène diffuse bien depuis l'espace des résistances vers l'espace process. La différence des pressions partielles de l'hydrogène sera le plus souvent telle que la pression partielle de l'hydrogène au sein du gaz contenu dans l'espace des résistances soit supérieure d'au moins 0,1 % et de préférence d'au moins 1 % à celle de l'hydrogène contenu dans le gaz process. La différence de pression absolue entre l'espace des résistances et l'espace process, ou surpression, sera de préférence telle que la pression dans l'espace des résistances soit supérieure d'au moins 0,1 % et le plus souvent d'au moins 1 % à la pression dans l'espace process. Il n'est pas nécessaire d'avoir une très grande surpression et le plus souvent la pression dans l'espace des résistances reste inférieure à 2 fois la pression dans l'espace process.

La figure 2 représente un détail d'un mode de réalisation de la zone de chauffage suivant l'invention. On utilise comme moyen de chauffage électrique des résistances (3) de forme cylindrique. Ces résistances comportent à chacune de leur extrémité des zones froides, et une partie de la zone centrale qui est la zone chaude représente par exemple environ 68 % de la longueur totale.

On réalise un réacteur de section rectangulaire, dont les parois sont constituées de béton réfractaire isolant (14) et par une armature métallique (15). On perce dans deux parois latérales opposées un trou circulaire, dans lequel on fait passer une gaine (4), par exemple en céramique, de diamètre double de celui de la résistance électrique (3). La gaine (4) est positionnée au moyen d'un système presse étoupe (16) agissant dans une gorge au niveau de l'armature métallique sur une tresse en matière réfractaire (17), par exemple une tresse en matière céramique. Le positionnement de la résistance (3) dans la gaine (4) est effectué au moyen de rondelles (18), par exemple en fibre céramique, comportant des orifices (23) permettant le passage du gaz contenant de l'hydrogène introduit dans le boîtier (11) par le conduit (12) dans l'espace des résistances (24).

La zone chaude de la résistance (3) est positionnée de façon à ce qu'elle ne pénètre pas dans l'orifice de passage à travers la paroi de béton isolant. Il n'est pas indispensable d'utiliser une tresse (17) au niveau du presse étoupe puisque celui-ci a, dans le cadre de l'invention, le rôle de moyen de positionnement et qu'il n'a pas pour but principal d'assurer une étanchéité aussi parfaite que possible entre l'intérieur et l'extérieur du réacteur. Ce presse étoupe peut d'ailleurs avantageusement être remplacé par un moyen plus simple de positionnement des gaines tel que par exemple des simples rondelles en matériau réfractaire.

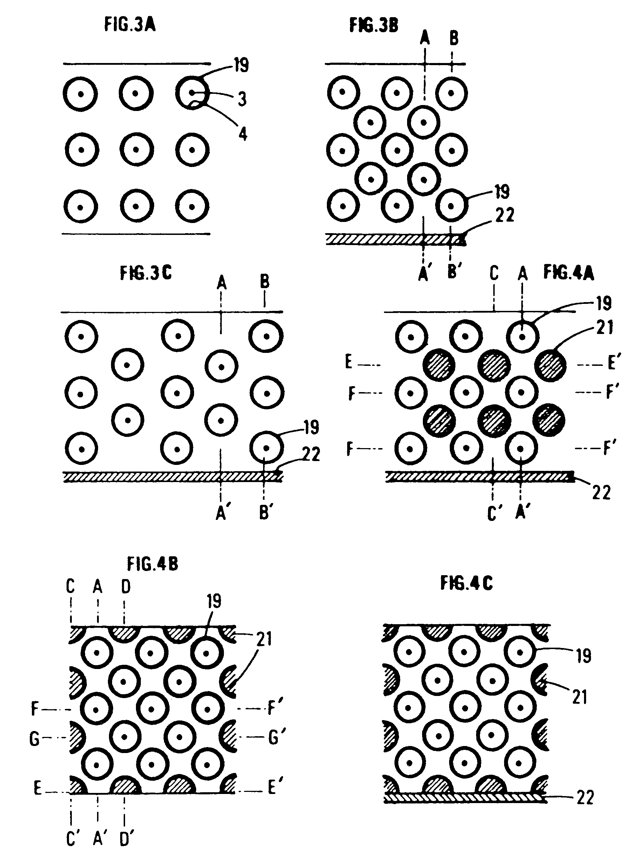

On dispose ainsi d'un certain nombre de résistances chauffantes gainées dans des parois, par exemple en matière céramique, par rangées horizontales successives, ces rangées étant de préférence alignées de façon à ce que, sur les parois latérales du four, elles forment un faisceau à pas carré ou rectangulaire. Un boîtier (11), dont dépassent seulement les extrémités des résistances et/ou leur alimentation électrique (6), est parcouru par un courant de gaz contenant de l'hydrogène. Les figures 3A, 3B, 3C, 4A, 4B et 4C représentent schématiquement une coupe longitudinale de 6 types de modules, utilisables pour la construction du four de pyrolyse selon l'invention, suivant un plan perpendiculaire à l'axe des éléments. Dans le cas des figures 3A, 3B, 3C ces modules ne comportent que des éléments chauffants(19). Dans le cas des figures 4A, 4B, 4C, ces modules comportent des éléments chauffants (19) et des pseudo éléments chauffants (21). Chaque module comportera le plus souvent de 2 à 30 de ces éléments et de préférence de 5 à 15 éléments. Chaque module comportera habituellement de 1 à 30 et le plus souvent de 5 à 11 éléments chauffants.

Le module schématisé sur la figure 3A comporte 3 rangées transverses AA' d'éléments chauffants. Par association de modules de ce type suivant une direction perpendiculaire aux rangées transverses (c'est-à-dire parallèle au sens d'écoulement des gaz), on pourra former une zone longitudinale comprenant 3 nappes d'éléments chauffants. On peut également associer des modules de ce type suivant deux directions perpendiculaires et former ainsi une zone longitudinale comportant par exemple 6 nappes d'éléments chauffants si on a associé les modules 2 par 2 suivant la direction perpendiculaire à AA'. Le module schématisé sur cette figure 3A est formé de motifs élémentaires comportant chacun une rangée transverse de 3 éléments chauffants. Les éléments constitutifs de ce module forment en projection horizontale un faisceau à pas carré.

Le module schématisé sur la figure 3B comporte 4 rangées transverses d'éléments chauffants. Ce module est formé de rangées transverses AA' comportant 3 éléments chauffants, séparées les unes des autres par des rangées transverses BB' de 2 éléments chauffants. Les éléments constitutifs de ce module forment en projection horizontale un faisceau à pas triangulaire. Le module schématisé sur cette figure 3B est formé de motifs élémentaires comportant chacun deux rangées transverses, l'une de 3 éléments chauffants et la suivante de 2 éléments chauffants. Ce module comporte en outre une paroi réfractaire (22).

Le module schématisé sur la figure 3C comporte 4 rangées transverses d'éléments chauffants. Ce module ne diffère du module schématisé sur la figure 3B que par le fait que la distance oblique entre les éléments est égale à la distance dans le sens transversal entre ces éléments et la distance dans le sens de circulation des gaz entre les éléments est supérieure à la distance dans le sens transversal.

L'emploi de modules tels que ceux schématisés sur la figure 3B et ceux schématisés sur la figure 3C, ayant une géométrie en quinconce avec des éléments situés à une distance p les uns des autres dans les sens d'écoulements des gaz et transversalement et à une distance oblique (suivant un axe sensiblement à 45 degré d'angle de l'axe AA' des rangées transverses), p/2 dans le cas du module schématisé sur la figure 3B et p dans le cas du module schématisé sur la figure 3C, permet de créer des zones a vitesse constante pour les gaz (module selon la figure 3B) et des zones à vitesse variable pour les gaz (module selon la figure 3C).

Le module schématisé sur la figure 4A comporte 3 rangées transverses AA' d'éléments chauffants et 3 rangées transverses CC' de pseudo éléments chauffants. Par association de modules de ce type suivant une direction perpendiculaire aux rangées transverses (c'est-à-dire parallèle au sens d'écoulement des gaz), on pourra former une zone longitudinale comprenant des nappes FF' d'éléments chauffants et des nappes EE' de pseudo éléments chauffants. Le module schématisé sur cette figure 4A est formé de motifs élémentaires comportant chacun deux rangées transverses, l'une de 3 éléments chauffants et la suivante de 2 pseudo éléments chauffants.

Le module schématisé sur la figure 4B comporte 7 rangées transverses d'éléments : 3 rangées transverses AA' d'éléments chauffants, 2 rangées transverses CC' de pseudo éléments chauffants et 2 rangées transverses DD' comportant des éléments chauffants et des pseudo éléments chauffants. Par association de modules de ce type suivant une direction perpendiculaire aux rangées transverses (c'est-à-dire parallèle au sens d'écoulement des gaz) et suivant une direction parallèle à ces rangées transverses, on pourra former une zone longitudinale comprenant des nappes FF' d'éléments chauffants, des nappes EE' de pseudo éléments chauffants et des nappes GG' comprenant des éléments chauffant et des pseudo éléments chauffants. Ce module comporte des éléments chauffants sensiblement cylindriques et les pseudo éléments chauffant situés sur la périphérie du module (sur les côtés du module) sont des éléments pleins qui ont, suivant leur position, sensiblement la forme d'un demi-cylindre ou sensiblement celle d'un quart de cylindre, de sorte que par juxtaposition des modules on forme des pseudo éléments, sensiblement cylindriques et ayant une section de surface sensiblement égale à celle de la section des éléments chauffants, ou demi-cylindriques pour ceux qui seront à proximité des parois de la zone longitudinale.

Le module schématisé sur la figure 4C ne diffère du module schématisé sur la figure 4B que par le fait qu'il comporte en outre une paroi réfractaire (22).

L'association de modules de type différent est tout à fait possible. Ainsi par exemple, on peut, dans le cas du choix d'une géométrie en quinconce des éléments formant une zone longitudinale, associer des modules tels que ceux schématisés sur les figures 3B et 3C.

### Exemple 1

On utilise un réacteur horizontal à trempe indirecte, de longueur totale de 6,1 mètre et de section rectangulaire de 1,4 x 2,89 m. Les moyens de chauffage de ce réacteur sont constitués par des résistances électriques en épingle, de marque KANTHAL, en bisiliciure de molybdène (MoSi2) de type SUPERKANTHAL ; ces résistances sont entourées de gaines en céramique, disposées concentriquement par rapport au centre du cercle englobant les résistances.

Ces gaines sont en carbure de silicium fabriqué par la société NORTON, elles sont de type KRYSTON et ont une porosité ouverte de 15 % en volume. Chaque gaine, fermée à une extrémité, entoure 2 résistances en épingle (figure 1B). Ces gaines sont disposées perpendiculairement au sens de circulation de la charge (verticalement), en nappes parallèles, et forment en projection perpendiculaire un faisceau à pas carré. La longueur de chaque branche de l'épingle de la résistance électrique est de 1,4 m et son diamètre est de 9 mm. Les gaines en céramique ont une longueur de 1,4 m, un diamètre extérieur de 150 mm et un diamètre intérieur de 130 mm ; la distance séparant deux gaines voisines est de 10 mm.

La première partie de la zone de chauffage, longue de 3,7 m, comprend 18 nappes de résistances, chaque nappe comprenant 23 gaines ; dans cette zone, la charge, préchauffée à 800 °C, est portée à 1200 °C. Cette zone est régulée thermiquement par l'intermédiaire de thermocouples disposés dans les espaces où circule la charge.

La deuxième partie de la zone de chauffage, adjacente à ladite première partie, est longue de 2,4 m ; elle est constituée de 18 nappes de 15 gaines, disposées de la même façon que dans la première partie de la zone de chauffage. Cette zone est constituée par 5 sections de chauffage, régulées indépendamment, permettant d'assurer le maintien de la température dans cette zone à 1200 °C plus ou moins 10 °C.

Les gaz effluents sont refroidis dans un premier temps à 800 °C par échange indirect avec les gaz de la charge ; d'autres échangeurs de température permettent ensuite d'abaisser leur température à 300 °C environ.

On utilise comme charge du méthane dilué avec de l'hydrogène dans un rapport volumique de 1 : 1. Ce mélange est préchauffé à 800 °C et craqué dans le réacteur décrit ci-avant. La pression absolue du mélange de gaz dans le réacteur est maintenue sensiblement constante et égale à 0,125 MPa. On introduit dans l'espace des résistances de l'hydrogène sensiblement pur de manière à obtenir et à maintenir dans cet espace une pression absolue sensiblement constante et égale à 0,130 MPa. Après refroidissement à température ambiante, pour 200 moles de mélange équivolumique de méthane et d'hydrogène, on obtient les quantités suivantes de produits principaux :

| PRODUITS | QUANTITES |
|---|---|
| H₂ | 143 moles |
| CH₄ | 70 moles |
| C₂H₂ | 6 moles |
| C₂H₄ | 4 moles |
| Benzène | 0,75 moles |
| Coke | 54 grammes |

### Exemple 2

On utilise un réacteur horizontal à trempe indirecte, de longueur totale de 4,31 mètre et de section rectangulaire de 1,4x2,94 m. Les moyens de chauffage de ce réacteur sont constitués par des résistances électriques en épingle, de marque KANTHAL, en bisiliciure de molybdène (MoSi₂) de type SUPERKANTHAL ; ces résistances sont entourées de gaines en céramique, disposées concentriquement par rapport au centre du cercle englobant les résistances.
Ces gaines sont en carbure de silicium fabriqué par la société NORTON, elles sont de type KRYSTON et ont une porosité ouverte de 15 % en volume. Chaque gaine, fermée à une extrémité, entoure 2 résistances en épingle (Figure 1B). Ces gaines sont disposées perpendiculairement au sens de circulation de la charge (verticalement), en nappes parallèles, et forment en projection perpendiculaire un faisceau à pas carré. La longueur de chaque branche de l'épingle de la résistance électrique est de 1,4 m et son diamètre est de 9 mm. Les gaines en céramique ont une longueur de 1,4 m, un diamètre extérieur de 150 mm et un diamètre intérieur de 130 mm ; les distances Eg et Et (Figure 1A) séparant deux gaines voisines sont de 10 mm. Le réacteur comprend 2 zones longitudinales, comprenant chacune 6 nappes d'éléments chauffants, séparées par une paroi en béton réfractaire à base d'alumine électrofondue. La distance Ee (Figure 1A) entre les gaines et la paroi ou dimension des passages est de 10 mm. Les parois ont dans leur partie la plus mince une épaisseur Ep (Figure 1A) de 15 mm. Le réacteur comporte ainsi 18 nappes de 27 éléments chauffants et 2 parois.

La première partie de la zone de chauffage, longue de 1,75 m, comprend 18 nappes de résistances, chaque nappe comprenant 11 gaines ; dans cette zone, la charge, préchauffée à 1000 °C, est portée à 1200 °C. Cette zone est régulée thermiquement par l'intermédiaire de thermocouples disposés dans les espaces où circule la charge.

La deuxième partie de la zone de chauffage, adjacente à ladite première partie, est longue de 2,56 m ; elle est constituée de 18 nappes de 16 gaines, disposées de la même façon que dans la première partie de la zone de chauffage. Cette zone est constituée par 3 sections de chauffage, régulées indépendamment, permettant d'assurer le maintien de la température dans cette zone à 1200 °C plus ou moins 10 °C.

Les gaz effluents sont refroidis dans un premier temps à 800 °C par échange indirect avec les gaz de la charge ; d'autres échangeurs de température permettent ensuite d'abaisser leur température à 300 °C environ.

On utilise comme charge du méthane dilué avec de l'hydrogène dans un rapport volumique de 1 : 1. Ce mélange est préchauffé à 1000 °C et craqué dans le réacteur décrit ci-avant. La pression absolue du mélange de gaz dans le réacteur est maintenue sensiblement constante et égale à 0,125 MPa. On introduit dans l'espace des résistances de l'hydrogène sensiblement pur de manière à obtenir et à maintenir dans cet espace une pression absolue sensiblement constante et égale à 0,130 MPa. Après refroidissement à température ambiante, pour 200 moles de mélange équivolumique de méthane et d'hydrogène, on obtient les quantités suivantes de produits principaux :

| PRODUITS | QUANTITES |
|---|---|
| H₂ | 142,5 moles |
| CH₄ | 70 moles |
| C₂H₂ | 6,3 moles |
| C₂H₄ | 4 moles |
| Benzène | 0,74 moles |
| Coke | 50 grammes |

## Revendications

1. Procédé de conversion thermique du méthane en hydrocarbures de poids moléculaire plus élevé dans une zone de réaction, de forme allongée selon une direction, comprenant une zone de chauffage et une zone de refroidissement faisant suite à ladite zone de chauffage et dans laquelle on refroidit les effluents de la zone de chauffage, selon lequel on fait circuler un mélange gazeux renfermant du méthane, dans ladite zone de chauffage, selon une direction d'écoulement sensiblement parallèle à la direction de la zone de réaction, caractérisé en ce que ladite zone de chauffage comporte, entre deux parois en matériau réfractaire, au moins une zone longitudinale, dans laquelle circule ledit mélange gazeux, ladite zone longitudinale comprenant une pluralité d'éléments, disposés en au moins deux nappes, sensiblement parallèles entre elles et sensiblement parallèles à la direction de la zone de réaction, lesdits éléments formant en projection transversale un faisceau à pas triangulaire, carré ou rectangulaire, l'une au moins de ces nappes comprenant une série de gaines à l'intérieur desquelles sont disposés des moyens de chauffage électrique formant ainsi une nappe d'éléments chauffants, lesdits moyens de chauffage étant ainsi isolés du contact direct avec le mélange gazeux renfermant du méthane, et lesdits éléments chauffants étant regroupés par sections successives transversales, comportant chacune au moins une rangée transverse d'éléments, lesdites sections étant sensiblement perpendiculaires à la direction de la zone de réaction, indépendantes entre elles et alimentées en énergie électrique de façon à déterminer au moins deux parties dans la zone de chauffage, la première partie permettant de porter la charge jusqu'à une température au plus égale à environ 1500 °C, et la deuxième partie, subséquente de la première partie, permettant de maintenir la charge à une température sensiblement égale à la température maximum à laquelle elle a été portée dans ladite première partie, et selon lequel on refroidit les effluents de la zone de chauffage par introduction dans la zone de refroidissement d'un fluide de refroidissement, puis on recueille lesdits hydrocarbures de poids moléculaires plus élevés à l'extrémité de la zone réactionnelle.

2. Procédé selon la revendication 1 dans lequel la zone longitudinale comporte au moins une nappe d'éléments chauffants et au moins une nappe d'éléments en matériau réfractaire formant une nappe de pseudo éléments chauffants.

3. Procédé selon la revendication 1 ou 2 dans lequel les éléments sont des éléments sensiblement cylindriques ayant tous un diamètre externe sensiblement égal et une hauteur sensiblement égale, et dans lequel ceux renfermant les moyens de chauffage et formant une nappe d'éléments chauffants sont des gaines cylindriques dont le diamètre interne est d'environ 1,2 à environ 8 fois le diamètre maximal du cercle englobant lesdits moyens de chauffage et les autres éléments sont des éléments creux tels que des gaines cylindriques ou des éléments cylindriques pleins.

4. Procédé selon l'une des revendications 1 à 3 dans lequel chaque paroi en matériau réfractaire séparant deux zones longitudinales adjacentes comporte au moins un moyen permettant d'équilibrer les pressions entre lesdites zones.

5. Procédé selon l'une des revendications 1 à 4 dans lequel les éléments chauffants comportent une gaine en matériau céramique.

6. Procédé selon l'une des revendications 1 à 5 dans lequel les moyens de chauffage électrique sont isolés du contact direct avec le mélange gazeux renfermant du méthane par des gaines dans lesquelles on introduit un gaz contenant de l'hydrogène, lesdites gaines ayant une perméabilité appropriée et le gaz étant introduit à l'intérieur desdites gaines à une pression telle qu'il y a diffusion, au moins en certains points, d'au moins une partie de l'hydrogène depuis l'intérieur desdites gaines vers l'extérieur desdites gaines.

7. Dispositif pour la mise en oeuvre du procédé selon l'une des revendications 1 à 6 comprenant un réacteur (1), de forme allongée selon un axe, de préférence à section carrée ou rectangulaire, comportant à une première extrémité des moyens d'alimentation en mélange gazeux, à l'extrémité opposée des moyens d'évacuation des effluents produits et entre ces deux extrémités des moyens d'alimentation en fluide de refroidissement, caractérisé en ce que ledit réacteur comprend dans une première partie (côté première extrémité) au moins une zone longitudinale (20) entre deux parois en matériau réfractaire sensiblement parallèles entre elles et sensiblement parallèles à l'axe du réacteur, ladite zone longitudinale comprenant une pluralité d'éléments, disposés en au moins deux nappes, sensiblement parallèles entre elles et sensiblement parallèles à l'axe du réacteur, l'une au moins de ces nappes comprenant une série de gaines (4) à l'intérieur desquelles sont disposés des moyens de chauffage électrique (3) formant ainsi une nappe d'éléments chauffants, lesdits éléments étant disposés de façon à définir entre eux et/ou entre les nappes qu'ils forment et/ou entre eux et les parois des espaces ou passages pour la circulation des mélanges gazeux et/ou des effluents, lesdits moyens de chauffage et lesdites gaines étant adaptés à chauffer lesdits passages par section transversales successives, indépendantes et sensiblement perpendiculaires à l'axe du réacteur, chaque section transversale comportant au moins une rangée transverse d'éléments et ledit réacteur comportant en outre des moyens d'asservissement et de modulation de chauffage reliés auxdits moyens de chauffage, et comportant dans une deuxième partie (8) (côté extrémité opposée) contiguë à la première partie des moyens de refroidissement (9) des effluents reliés auxdits moyens d'alimentation en fluide de refroidissement.

8. Dispositif selon la revendication 7 dans lequel chaque zone longitudinale est formée par la juxtaposition d'une série de modules constitutifs, de section carrée ou rectangulaire, comprenant chacun au moins deux éléments formant une rangée transverse, dont l'un au moins est formé d'une gaine (4), à l'intérieur de laquelle est disposé un moyen de chauffage électrique (3), et constitue un élément chauffant (19), lesdits éléments étant disposés de façon à définir entre eux et/ou entre eux et les parois de ladite zone des espaces ou passages pour la circulation des mélanges gazeux et/ou des effluents, et lesdits modules étant juxtaposés de manière à ce que les éléments forment, entre deux parois en matériau réfractaire sensiblement parallèles entre elles et sensiblement parallèles à l'axe du réacteur, au moins deux nappes, sensiblement parallèles entre elles et sensiblement parallèles à l'axe du réacteur.

9. Dispositif selon la revendications 8 dans lequel le module constitutif est choisi parmi les modules constitutifs comportant au moins deux rangées transverses de deux ou trois éléments chauffants disposés de manière à ce que la juxtaposition de ces modules permette d'obtenir au moins deux nappes d'éléments chauffants, lesdites nappes étant perpendiculaires à l'axe du réacteur et lesdits éléments formant en projection transversale un faisceau à pas triangulaire, carré ou rectangulaire.

10. Dispositif selon la revendication 8 dans lequel le module constitutif comporte au moins deux rangées transverses dont l'une au moins est formée d'éléments chauffants et dont au moins une autre contigue à une rangée d'éléments chauffants est formée de pseudo éléments chauffants en matériau réfractaire, lesdits éléments étant disposés de manière à ce que la juxtaposition de ces modules permette d'obtenir au moins deux nappes d'éléments, lesdites nappes étant perpendiculaires à l'axe du réacteur et lesdits éléments formant en projection transversale un faisceau à pas triangulaire, carré ou rectangulaire.

11. Dispositif selon la revendication 10 dans lequel le module constitutif comporte des pseudo éléments chauffants sur sa périphérie, lesdits pseudo éléments chauffants ayant un section telle que par juxtaposition des modules, ceux situés au niveau de l'arête par laquelle lesdits modules sont juxtaposés forment des pseudo éléments ayant sensiblement la même section que les éléments chauffants, ceux contigus à un côté ont une section inférieure à la section des éléments chauffants et de préférence une section égale à environ la moitié de la section desdits éléments chauffants et égale à environ le quart pour ceux contigus à deux côtés du module.

12. Dispositif selon l'une des revendications 7 à 11 dans lequel le réacteur comporte au moins deux zones longitudinales formées par la juxtaposition dans le sens longitudinal et dans le sens transversal d'une série de modules comportant chacun au moins une paroi longitudinale en matériau réfractaire.

13. Dispositif selon l'une des revendications 7 à 12 dans lequel chaque paroi en matériau réfractaire séparant deux zones longitudinales adjacentes comporte au moins un moyen permettant l'équilibrage des pressions entre les deux zones.

## Claims

1. A process for the thermal conversion of methane into hydrocarbons of higher molecular weight in a reaction zone which is elongate in one direction, comprising a heating zone and a cooling zone following on from the said heating zone and in which the effluents from the heating zone are cooled, a gaseous mixture containing methane being caused to circulate in the said heating zone in a direction of flow which is substantially parallel with the direction of the reaction zone, characterised in that the said heating zone comprises between two walls of refractory material, at least one longitudinal zone in which the said gaseous mixture circulates, the said longitudinal zone comprising a plurality of elements disposed in at least two layers which are substantially parallel inter se and substantially parallel with the direction of the reaction zone, the said elements forming in transverse projection a cluster of triangular, square or rectangular pitch, at least one of these layers comprising a series of sheaths inside which there are electric heating means, so forming a layer of heating elements, the said heating means thus being insulated from direct contact with the gaseous mixture containing the methane and the said heating elements being grouped in successive transverse sections each comprising at least one transverse row of elements, the said sections being perpendicular to the direction of the reaction zone, independent of one another and supplied with electrical energy in such a way as to define at least two parts in the heating zone, the first part making it possible to raise the charge to a temperature at least equal to approx. 1500°C and the second part, following on from the first part, making it possible to maintain the charge at a temperature substantially equal to the maximum temperature to which it was raised in the first part, and according to which the effluent from the heating zone is cooled by introduction into the cooling zone of a cooling fluid after which the said hydrocarbons of higher molecular weight are collected at the end of the reaction zone.

2. A process according to claim 1 in which the longitudinal zone comprises at least one layer of heating elements and at least one layer of elements of refractory material which form a layer of pseudo heating elements.

3. A process according to claim 1 or 2 in which the elements are substantially cylindrical elements all having a substantially equal outside diameter and a substantially equal height, and in which those containing the heating means and forming a layer of heating elements are cylindrical sheaths with an inside diameter of approx. 1.2 to approx. 8 times the maximum diameter of the circle embracing the said heating means and the other elements are hollow elements such as cylindrical sheaths or solid cylindrical elements.

4. A process according to one of claims 1 to 3 in which each wall of refractory material separating two adjacent longitudinal zones comprises at least one means of ensuring a balancing of the pressures between the said zones.

5. A process according to one of claims 1 to 4 in which the heating elements comprise a sheath of ceramic material.

6. A process according to one of claims 1 to 5 in which the electric heating means are insulated from direct contact with the gaseous mixture containing methane by sheaths into which a gas containing hydrogen is introduced, the said sheaths having a suitable permeability and the gas being introduced into the interior of the said sheaths at such a pressure that at least at certain points there is a diffusion of at least a part of the hydrogen from the interior of the said sheaths to the exterior of the said sheaths.

7. An apparatus for carrying out the method according to one of claims 1 to 6, comprising a reactor (1) of elongate form, according to an axis of preferably square or rectangular cross-section, comprising at a first end means of supplying a gaseous mixture and at the opposite end means of discharging the effluent produced and, between these two ends, means of supplying a cooling medium, characterised in that the said reactor comprises in a first part (the same side as the first end) at least one longitudinal zone (20) between two walls of refractory material, which are substantially parallel with each other and substantially parallel with the axis of the reactor, the said longitudinal zone comprising a plurality of elements, disposed in at least two layers, substantially parallel inter se and substantially perpendicular to the axis of the reactor, at least one of these layers comprising a series of sheaths (4) inside which there are electric heating means (3) which thus form a layer of heating elements, the said elements being disposed in such a way as to define between them and/or between the layers which they form and/or between them and the walls of the spaces or passages for circulation of the gaseous mixtures and/or effluents, the said heating means and the said sheaths being adapted to heat the said passages in successive independent transverse sections which are substantially at right-angles to the axis of the reactor, each transverse section comprising at least one transverse row of elements while the said reactor comprises in addition means for the automatic control and modulation of the heating and which are connected to the said heating means, and comprising in a second part (8) (the side of the opposite end) contiguous to the first part, means (9) of cooling the effluent and connected to the said cooling medium supply means.

8. An apparatus according to claim 7 in which each longitudinal zone is formed by the juxtaposition of a series of constituent modules of square or rectangular cross-section, each comprising at least two elements forming a transverse row of which at least one is formed by a sheath (4) inside which there is an electric heating means (3), and constiutes a heating element (19), the said elements being disposed in such a way as to define between them and/or between them and the walls of the said zone spaces or passages for the circulation of gaseous mixtures and/or effluents, and the said modules being juxtaposed in such a way that the elements form between two walls of refractory material which are substantially parallel inter se and substantially parallel with the axis of the reactor, at least two layers, substantially parallel inter se and substantially parallel with the axis of the reactor.

9. An apparatus according to claim 8 in which the constituent module is chosen from among the constituent modules comprising at least two transverse rows of two or three heating elements which are so disposed that the juxtaposition of these modules makes it possible to obtain at least two layers of heating elements, the said layers being perpendicular to the axis of the reactor and the said elements forming in transverse projection a cluster of triangular, square or rectangular configuration.

10. An apparatus according to claim 8 in which the constituent module comprises at least two transverse rows of which at least one is formed by heating elements and of which at least one other, contiguous upon a row of heating elements, is formed by pseudo heating elements of refractory material, the said elements being disposed in such a way that the juxtaposition of these modules makes it possible to obtain at least two layers of elements, the said layers being perpendicular to the axis of the reactor and the said elements forming in transverse projection a cluster of triangular, square or rectangular configuration.

11. An apparatus according to claim 10 in which the constituent module comprises pseudo heating elements on its periphery, the said pseudo heating elements having a cross-section such that by juxtaposition of the modules, those situated at the level of the edge by which the said modules are juxtaposed form pseudo elements having substantially the same cross-section as the heating elements, those contiguous upon one side have a cross-section smaller than the cross-section of the heating elements and preferably a cross-section equal to approx. half the cross-section of the said heating elements and equal to approx. one quarter in the case of those contiguous upon two sides of the module.

12. An apparatus according to one of claims 7 to 11, in which the reactor comprises at least two longitudinal zones formed by the juxtaposition in the longitudinal sense and in the transverse sense of a series of modules each comprising at least one longitudinal wall of refractory material.

13. An apparatus according to one of claims 7 to 12, in which each wall of refractory material separating two adjacent longitudinal zones comprises at least one means permitting balancing of the pressures between the two zones.

## Patentansprüche

1. Verfahren zur thermischen Umwandlung von Methan in Kohlenwasserstoffe von höherer Molmasse in einer Reaktionszone, die eine Heizzone und eine Abkühlzone, die sich an die besagte Heizzone anschließt und in der die Austragsstoffe der Heizzone abgekühlt werden, umfaßt, die in eine Richtung verlängert ist, entlang der man in der besagten Heizzone ein Gasgemisch, das Methan enthält, entlang einer Strömungsrichtung, die etwa parallel der Ausrichtung der Reaktionszone ist, strömen läßt, dadurch gekennzeichnet, daß die besagte Heizzone zwischen zwei Wänden aus feuerfestem Material mindestens eine longitudinale Zone umfaßt, in der das besagte Gasgemisch strömt, die besagte longitudinale Zone umfaßt eine Vielzahl von Elementen, die in mindestens zwei Lagen angeordnet sind, die zueinander etwa parallel und zur Richtung der Reaktionszone etwa parallel stehen, wobei die besagten Elemente in der Seitenansicht ein dreieckiges, quadratisches oder rechteckiges Bündel bilden und mindestens eine dieser Schichten eine Reihe von Mänteln umfaßt, in deren Innerem die Mittel zur elektrischen Beheizung angeordnet sind, die auf diese Weise eine Lage von Heizelementen bilden, wobei die besagten Heizmittel auf diese Weise vom direkten Kontakt mit dem das Methan enthaltenden Gasgemisch isoliert sind und die besagten Heizelemente durch aufeinanderfolgende transversale Abschnitte gegliedert sind, von denen jeder mindestens eine Querreihe von Elementen umfaßt, die besagten Abschnitte etwa senkrecht zur Richtung der Reaktionszone stehen, voneinander unabhängig sind und mit elektrischer Energie versorgt werden, so daß sie mindestens zwei Teile in der Reaktionszone begrenzen, wobei es der erste Teil erlaubt, den Einsatzstoff auf eine Temperatur von mehr als ungefähr 1500 °C zu bringen und der zweite, auf den ersten Teil folgende, Teil es erlaubt, den Einsatzstoff auf einer Temperatur zu halten, die etwa gleich der Höchsttemperatur ist, auf die er im besagten ersten Teil gebracht wurde, und entlang dem die Austragsstoffe der Heizzone durch Einleitung in die Abkühlzone mit einer Kühlflüssigkeit abgekühlt werden, und schließlich die besagten Kohlenwasserstoffe höherer Molmassen am Ende der Reaktionszone erhalten werden.

2. Verfahren nach Anspruch 1, bei dem die longitudinale Zone mindestens eine Lage Heizelemente und mindestens eine Lage von Elementen aus feuerfestem Material, die eine Lage Pseudoheizelemente bilden, umfaßt.

3. Verfahren nach Anspruch 1 oder 2, bei dem die Elemente etwa zylindrische Elemente sind, die alle einen etwa gleichen Außendurchmesser und eine etwa gleiche Höhe haben und bei denen diejenigen, welche die Heizmittel einschließen und eine Lage von Heizelementen bilden, zylindrische Mäntel sind, deren Innendurchmesser zwischen 1,2 und 8 mal dem maximalen Durchmesser des Kreises beträgt, der die besagten Heizmittel umgibt, und die anderen Elemente hohle Elemente wie zum Beispiel zylindrische Mäntel oder massive zylindrische Elemente sind.

4. Verfahren nach einem der Ansprüche 1 bis 3, bei dem jede Wand aus feuerfestem Material, welche die beiden longitudinalen benachbarten Zonen abtrennt, mindestens ein Mittel umfaßt, das es erlaubt, den Druck zwischen den besagten Zonen auszugleichen.

5. Verfahren nach einem der Ansprüche 1 bis 4, bei dem die Heizelemente einen Mantel aus keramischem Material umfassen.

6. Verfahren nach einem der Ansprüche 1 bis 5, bei dem die elektrischen Heizmittel vom direkten Kontakt mit dem das Methan enthaltenden Gasgemisch durch Mäntel isoliert sind, in die ein Wasserstoff enthaltendes Gas eingeleitet wird, die besagten Mäntel haben dabei eine geeignete Durchlässigkeit und das Gas wird ins Innere der besagten Mäntel unter einem Druck eingeleitet, unter dem zumindest an bestimmten Punkten die Diffusion von mindestens einem Teil des Wasserstoffs vom Inneren der besagten Mäntel zum Äußeren der besagten Mäntel stattfindet.

7. Vorrichtung zur Durchführung des Verfahrens nach einem der Ansprüche 1 bis 6, die einen Reaktor (1) umfaßt, der entlang einer Achse verlängert ist und vorzugsweise einen quadratischen oder rechteckigen Querschnitt hat, der an einem ersten Ende Mittel zur Zuführung eines gasförmigen Gemischs, am gegenüberliegenden Ende Mittel zur Abführung der gebildeten Austragsstoffe und zwischen diesen beiden Enden Mittel zur Zuführung von Kühlflüssigkeit umfaßt, dadurch gekennzeichnet, daß der besagte Reaktor in einem ersten Teil (auf der Seite des ersten Endes) mindestens einen longitudinalen Bereich (20) zwischen zwei Wänden aus feuerfestem Material umfaßt, die zueinander etwa parallel und zur Achse des Reaktors etwa parallel sind, wobei die besagte longitudinale Zone eine Vielzahl von Elementen umfaßt, die in mindestens zwei zueinander etwa parallelen und zur Achse des Reaktors etwa senkrechten Lagen angeordnet sind, wobei mindestens eine dieser Lagen eine Reihe von Mänteln (4) umfaßt, in deren Innerem die Mittel (3) zur elektrischen Beheizung angeordnet sind, die auf diese Weise eine Lage von Heizelementen bilden; diese besagten Elemente sind dabei so angeordnet, daß sie zwischen sich und/oder zwischen den Lagen, die sie bilden und/oder zwischen sich und den Wänden Räume oder Durchlässe zur Strömung des Gasgemisches und/oder der Austragsstoffe definieren, die besagten Heizmittel und die besagten Mäntel sind geeignet, die besagten Durchlässe in aufeinanderfolgenden transversalen Bereichen, unabhängig von der und annähernd senkrecht zur Achse des Reaktors, zu beheizen, wobei jeder transversale Bereich mindestens eine Querreihe von Elementen besitzt und der besagte Reaktor darüber hinaus mindestens Mittel zur Regelung und zur Modulation der Beheizung umfaßt, die mit den besagten Heizmitteln verbunden sind, und in einem zweiten Teil (8) (auf der Seite des gegenüberliegenden Endes), der an den ersten Teil angrenzt, Mittel zum Abkühlen (9) der Austragsstoffe umfaßt, die mit den besagten Mitteln zur Zuführung der Abkühlflüssigkeit verbunden sind.

8. Vorrichtung nach Anspruch 7, bei dem jede longitudinale Zone durch Aneinanderbauen einer Reihe von Basismodulen mit quadratischem oder rechteckigem Querschnitt gebildet wird, von denen jedes mindestens zwei eine Querreihe bildende Elemente besitzt, von denen mindestens eines von einem Mantel (4) gebildet wird, in dessen Innerem sich ein Mittel (3) zur elektrischen Beheizung befindet und ein Heizelement (19) darstellt, wobei die besagten Elemente so angeordnet sind, daß sie zwischen sich und/oder zwischen sich und den Wänden der besagten Zone Räume oder Durchlässe zur Strömung der Gasgemische und/oder der Austragsstoffe definieren, und die besagten Module so aneinandergereiht sind, daß die Elemente zwischen zwei zueinander und zur Achse des Reaktors etwa parallelen Wänden aus feuerfestem Material mindestens zwei zueinander und zur Achse des Reaktors etwa parallele Lagen bilden.

9. Vorrichtung nach Anspruch 8, in der das Basismodul unter denjenigen Basismodulen ausgewählt wird, die mindestens zwei Querreihen aus zwei oder drei Heizelementen umfassen, die so angeordnet sind, daß das Aneinanderbauen dieser Module es erlaubt, mindestens zwei Lagen von Heizelementen zu erhalten, wobei die besagten Lagen senkrecht zur Achse des Reaktors stehen und die besagten Elemente in der Seitenansicht ein dreieckiges, quadratisches oder rechteckiges Bündel bilden.

10. Vorrichtung nach Anspruch 8, bei der das Basismodul mindestens zwei Querreihen umfaßt, von denen mindestens eine von Heizelementen gebildet wird und von denen mindestens eine andere, direkt an eine Lage aus Heizelementen angrenzende, von Pseudoheizelementen aus feuerfestem Material gebildet wird, die besagten Elemente sind so angeordnet, daß das Aneinanderbauen dieser Module es erlaubt, mindestens zwei Lagen von Elementen zu erhalten, wobei die besagten Lagen senkrecht zur Achse des Reaktors stehen und die besagten Elemente in der Seitenansicht ein dreieckiges, quadratisches oder rechteckiges Bündel bilden.

11. Vorrichtung nach Anspruch 10, bei der das Basismodul Pseudoheizelemente am äußeren Bereich umfaßt, wobei die Pseudoheizelemente einen solchen Querschnitt besitzen, daß beim Aneinanderbauen der Module diejenigen, die sich im Bereich der Kante befinden, über welche die besagten Module aneinandergebaut werden, Pseudoelemente bilden, die etwa den gleichen Querschnitt haben wie die Heizelemente, diejenigen, die an einer Seite angrenzen, haben einen Querschnitt kleiner als der Querschnitt der Heizelemente und vorzugsweise einen Querschnitt von ungefähr der Hälfte des Querschnittes der besagten Heizelemente und von ungefähr einem Viertel bei solchen, die an zwei Seiten des Moduls angrenzen.

12. Vorrichtung nach einem der Ansprüche 7 bis 11, bei welcher der Reaktor mindestens zwei longitudinale Zonen umfaßt, die durch Aneinanderbauen in Längsrichtung und in Querrichtung einer Reihe von Modulen gebildet werden, von denen jedes mindestens eine Längswand aus feuerfestem Material umfaßt.

13. Vorrichtung nach einem der Ansprüche 7 bis 12, bei der jede Wand aus feuertestem Material, die zwei aneinandergrenzende longitudinale Zonen trennt, mindestens ein Mittel umfaßt, das den Druckausgleich zwischen den beiden Zonen erlaubt.
